(19) Europäisches Patentamt
European Patent Office
Office européen des brevets

(11) Veröffentlichungsnummer: **0 169 521**
**B1**

(12) # EUROPÄISCHE PATENTSCHRIFT

(45) Veröffentlichungstag der Patentschrift: **16.05.90**

(21) Anmeldenummer: **85109120.7**

(22) Anmeldetag: **22.07.85**

(51) Int. Cl.$^5$: **C 07 C 251/34,**
C 07 C 251/50, A 01 N 35/10

(54) **Cyclohexenonderivate, Verfahren zur ihrer Herstellung und ihre Verwendung zur Bekämpfung unerwünschten Pflanzenwuchses.**

(30) Priorität: **27.07.84 DE 3427695**
**14.09.84 DE 3433767**

(43) Veröffentlichungstag der Anmeldung:
**29.01.86 Patentblatt 86/05**

(45) Bekanntmachung des Hinweises auf die Patenterteilung:
**16.05.90 Patentblatt 90/20**

(84) Benannte Vertragsstaaten:
**BE CH DE FR GB IT LI NL**

(56) Entgegenhaltungen:
**EP-A-0 085 529**
**DE-A-3 248 554**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Keil, Michael, Dr.**
**Fontanestrasse 4**
**D-6713 Freinsheim (DE)**
Erfinder: **Schirmer, Ulrich, Dr.**
**Berghalde 79**
**D-6900 Heidelberg (DE)**
Erfinder: **Jahn, Dieter, Dr.**
**Burgunder Weg 8**
**D-6803 Edingen-Neckarhausen (DE)**
Erfinder: **Becker, Rainer, Dr.**
**Im Haseneck 22**
**D-6702 Bad Duerkheim (DE)**
Erfinder: **Wuerzer, Bruno, Dr.**
**Ruedigerstrasse 13**
**D-6701 Otterstadt (DE)**
Erfinder: **Meyer, Norbert, Dr.**
**Dossenheimer Weg 22**
**D-6802 Ladenburg (DE)**

**EP 0 169 521 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft Cyclhexenonderivate, Verfahren zu ihrer Herstellung, Herbizide, die diese Verbindungen als Wirkstoffe enthalten, sowie ein Verfahren zur Bekämpfung von unerwünschtem Pflanzenwuchs.

Es ist bekannt, Cyclohexenonderivate zur Bekämpfung von unerwünschten Gräsern in breitblättrigen Kulturen zu verwenden (DE—A—2 439 104). Außerdem ist aus der DE—A—3 248 554 und der EP—A—85 529 bekannt, daß Cyclohexen-1-on-derivative, die in 3-Stellung einen para-substiuierten Phenylring tragen, grasartige Unkräuter in Mais und Weizen bakämpfen.

Es wurde gefunden, daß Cyclohexanonderivate der Formel I

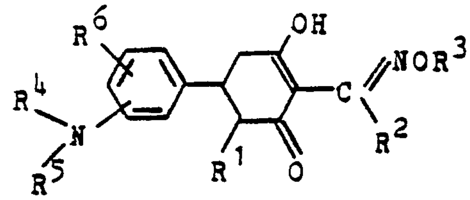

(I),

in der die Substituenten folgende Bedeutung haben:

$R^1$ Wasserstoff, Methoxycarbonyl, Ethoxycarbonyl oder Methyl

$R^2$ Alkyl mit 1 bis 4 C-Atomen,

$R^3$ Alkyl mit 1 bis 4 C-Atomen, Alkenyl mit 3 oder 4 C-Atomen, Halogen-alkenyl mit 3 oder 4 C-Atomen und 1 bis 3 Halogensubstituenten oder Propargyl,

$R^4$ Wasserstoff oder Alkyl 1 bis 4 C-Atomen,

$R^5$ aliphatisches Acyl mit 2 bis 8 C-Atomen, Formyl, Cycloalkylcarbonyl mit 4 bis 7 C-Atomen, Methoxylalkylcarbonyl, unsubstituiertes oder durch Nitro, Halogen, Alkyl, Alkoxy oder Haloalkyl substituiertes Benzoyl und

$R^6$ Alkyl, Halogen, Hydroxy oder Alkoxy,

und Salze dieser Verbindungen eine gute herbizide Wirkung, vorzugsweise gegen Arten aus der Familie der Gräser (Gramineen), besitzen. Sie sind verträglich und somit selektiv in breitblättrigen Kulturen sowie in monokotylen Gewäschen, welche nicht zu den Gramineen zählen. Ferner sind Verbindungen darunter, welche sich auch in Gramineenkulturen, wie Weizen und Reis, selektiv verhalten und gleichzeitig unerwünschte Gräser bekämpfen.

Die Cyclohexenonderivate der Formel I können in mehreren Formen auftreten, die alle vom Patentanspruch umfaßt werden:

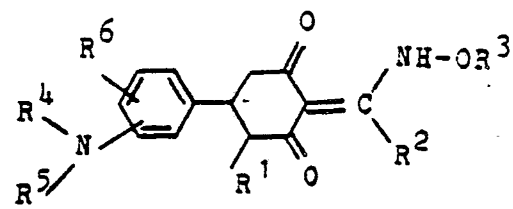

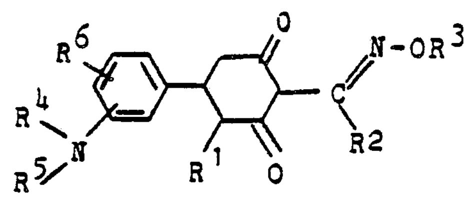

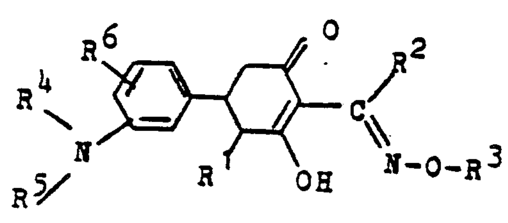

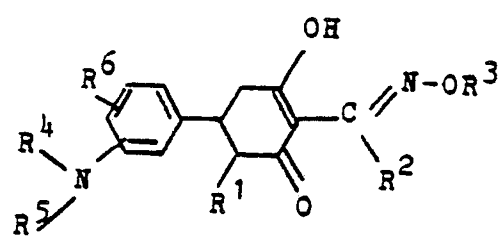

In der Formel I steht $R^1$ für Wasserstoff, Methoxycarbonyl, Ethoxycarbonyl oder Methyl, wobei Wasserstoff bevorzugt ist.

$R^2$ in Formel I steht für unverzweigte oder verzweigte Alkylreste mit 1 bis 4 C-Atomen, d.h. für Methyl, Ethyl, n-Propyl, i-Propyl, n-Butyl, sec-Butyl, i-Butyl, tert-Butyl. Alkylreste mit 2 oder 3 C-Atomen sind bevorzugt.

Reste für $R^3$ in Formel I sind Propargyl, Alkyl mit 1 bis 4 C-Atomen, Alkenyl mit 3 oder 4 C-Atomen oder Halogenalkenyl mit 3 oder 4 C-Atomen, das bis zu drei Halogensubstituenten enthalten kann, vorzugsweise Chloralkenyl, beispielsweise Methyl, Ethyl, n-Propyl, i-Propyl, n-Butyl, sec-Butyl, i-Butyl, tert-Butyl, Allyl, 1-Chlorprop-1-en-3-yl, 2-Chlor-prop-1-en-3-yl, 1,3-Dichlorprop-1-en-3-yl, 1,1,2-Trichlorprop-1-en-3-yl.

Reste für $R^4$ in Formel I sind Wasserstoff oder Alkyl mit 1 bis 4 C-Atomen, d.h. Methyl, Ethyl, Propyl, Isopropyl, n-Butyl, sec-Butyl, i-Butyl, tert-Butyl, wobei Wasserstoff bevorzugt ist.

$R^5$ in Formel I steht für aliphatisches Acyl mit 2 bis 8 C-Atomen, Formyl, Cycloalkylcarbonyl mit 4 bis 7 C-Atomen, Methoxyalkylcarbonyl mit 3 bis 7 C-Atomen, gegebenenfalls durch Nitro, Halogen, Alkyl, Alkoxy oder Haloalkyl substituiertes Benzoyl, wobei aliphatisches Acyl mit 2 bis 8 C-Atomen und gegebenenfalls durch Nitro, Halogen, Alkyl, Alkoxy oder Haloalkyl substituiertes Benzoyl bervorzugt ist. Besonders bevorzugt sind Wasserstoff, Acetyl und Benzoyl.

$R^6$ in Formel I steht in ortho-, meta- oder para-Position und bedeutet Halogen, Alkyl oder Alkoxy, jeweils mit bis zu 4 Kohlenstoffatomen, wobei Halogen, insbesondere Fluor oder Chlor bevorzugt ist.

Die Alkylgruppen für $R^5$ und $R^6$ sowie die Alkyl- und Alkoxygruppen in den für $R^5$ und $R^6$ angegebenen Resten können unverzweigt oder verzweigt sein und haben, soweit nichts anderes angegeben ist, 1 bis 4 C-Atome, d.h. für Alkyl oder Alkoxy kommen Methyl, Ethyl, n-Propyl, i-Propyl, n-Butyl, sec-Butyl, i-Butyl, tert-Butyl, Methoxy, Ethoxy, n-Propoxy, i-Propoxy, n-Butoxy, i-Butoxy, sec-Butoxy und tert-Butoxy in Betracht.

Beispiele für $R^5$ sind Formyl, Acetyl, Propionyl, Butyryl, Valeryl, Cyclopropylcarbonyl, Cyclopentylcarbonyl, Cyclohexylcarbonyl, Methoxyacetyl, 2-Methoxypropionyl, 3-Methoxypropionyl, Benzoyl, 3-Nitrobenzoyl, 2-Chlorbenzoyl, 4-Methylbenzoyl, 4-Methoxybenzoyl.

Beispiele für $R^6$ sind Methyl, Ethyl, n-Propyl, n-Butyl, i-Propyl, sec-Butyl, i-Butyl, tert-Butyl, Fluor, Chlor, Brom, Jod, Hydroxy, Methoxy, Ethoxy, n-Propoxy, i-Propoxy, n-Butoxy, i-Butoxy, sec-Butoxy, tert-Butoxy.

Als Salze der Verbindungen der Formel I kommen landwirtschaftlich brauchbare Salze, beispielsweise die Alkalimetallsalze, insbesondere die Kalium- oder Natriumsalze, Erdalkalimetallsalze, insbesondere Calcium-, Magnesium- oder Bariumsalze sowie Mangan-, Kupfer-, Zink- oder Eisensalze und Ammonium- und Phosphoniumsalze, beispielsweise Alkylammonium-, Dialkylammonium-, Trialkyl- oder Tetraalkylammoniumsalze, Benzyltrialkylammoniumsalze, Triphenylphosphoniumsalze, Trialkylsulfoniumsalze oder Trialkylsulfoxonsalze in Betract.

Die herbizid wirksamen Cyclohexenonderivate der Formel I können durch Umsetzung von Tricarbonylverbindungen der Formel II

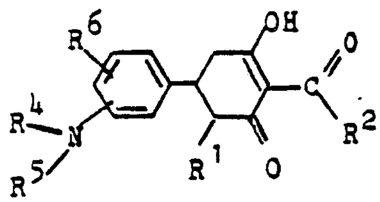

(II),

mit Ammoniumverbindungen $R^3O—NH_3Y$, in der $R^3$ die obengenannten Bedeutungen hat und Y ein Anion bedeutet, erhalten werden.

Man führt die Reaktion zweckmäßigerweise in heterogener Phase in einem inerten Verdünnungsmittel bei einer Temperatur zwischen 0 und 80°C oder zwischen 0°C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Außerdem können auch organische Basen, wie Pyridin oder tertiäre Amine, Verwendung finden.

Die Umsetzung verläuft besonders gut in einem pH-Bereich von 2 bis 9, insbesondere von 4,5 bis 5,5. Die Einstellung des pH-Bereiches erfolgt zweckmäßigerweise durch Zusatz von Acetaten, beispielsweise Alkalimetallacetaten, insbesondere von Natrium- oder Kaliumacetat oder einer Mischung aus beiden Salzen, Alkalimetallacetate werden beispielsweise in Mengen von 0,5 bis 2 Mol, bezogen auf die Ammoniumverbindung der Formel $R^3O—NH_3Y$, zugesetzt.

Als Lösungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid und Abdestillieren des Lösungsmittel unter vermindertem Druck isoliert werden.

Die Verbindungen der Formel I können außerdem durch Umsetzen der Tricarbonylverbindungen der Formel II mit Hydroxylamien der Formel $R^3O—NH_2$, in der $R^3$ die obengenannten Bedeutungen hat, in inerten Verdünnungsmitteln bei einer Temperatur zwischen 0°C und dem Siedepunkt des Reaktionsgemisches, insbesondere zwischen 15 und 70°C, erhalten werden. Gegebenenfalls kann das Hydroxylamin als wäßrige Lösung eingesetzt werden.

Geeignete Lösungsmittel für diese Umsetzung sind beispeilsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Alkalimetallsalze der Verbindungen der Formel I können durch Behandeln dieser Verbindungen mit Natrium- oder Kaliumhydroxid in wäßriger Lösung oder in einem organischen Lösungsmittel, wie

Methanol, Ethanol, Aceton, erhalten werden. Auch Natrium- und Kaliumalkoholate können als Basen dienen.

Die anderen Metallsalze, z.B. die Mangan-, Kupfer-, Zink-, Eisen- Calcium, Magnesium- und Bariumsalze können aus den Natriumsalzen durch Reaktion mit den entsprechenden Metallchloriden in wäßriger Lösung hergestellt werden. Ammonium-, Phosphonium-, Sulfonium- und Sulfoxoniumsalze können durch Umsetzung von verbindungen der Formel I mit Ammonium-, Phosphonium-, Sulfonium- oder Sulfoxoniumhydroxiden, gegebenenfalls in wäßriger Lösung, hergestellt werden.

Die neuen Tricarbonylverbindungen der Formel II können aus Cyclohexan-1,3-dionen der Formel

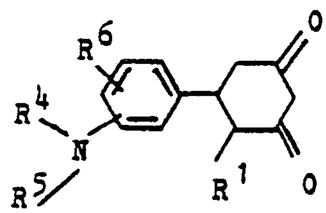

(VII),

in der $R^1$, $R^4$, $R^5$ und $R^6$ die oben genannte Bedeutung besitzen, nach literaturbekannten Methoden (Tetrahedron Letters, *29,* 2491 (1975)) hergestellt werden.

Es ist auch möglich, die neuen Verbindungen der Formel II über die Zwischenstufe der Enoleser, die bei der Acylierung von Verbindungen der Formel VII eventuell als Isomerengemische anfallen und in Gegenwart von Imidazol- oder Pyridinderivaten umgelagert werden (JP—A—63 052/1979), herzustellen.

Wie aus dem vorausgehenden ersichtlich, dienen die neuen Tricarbonylverbindungen der Formel II als wertvolle Zwischenprodukte für die Herstellung der herbizid wirksamen Verbindungen der Formel I.

Zu den Verbindungen der Formel II gelangt man wiederum nach literatur- bekannten Verfahren, wie dies auch folgendem Schema hervorgeht:

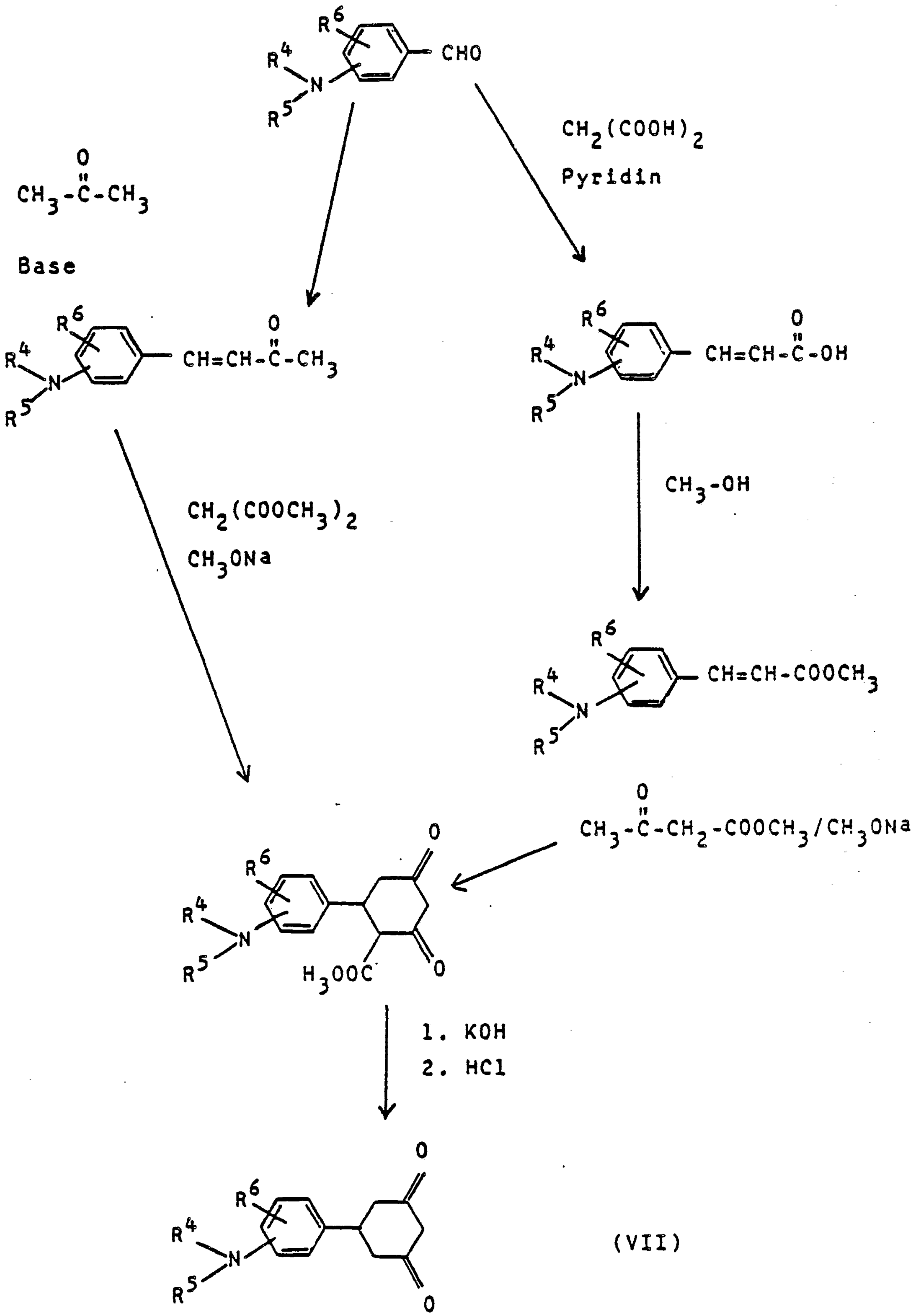
CHO
CH2(COOH)2
Pyridin
Base
CH=CH-C-CH3
CH=CH-C-OH
CH3-OH
CH2(COOCH3)2
CH3ONa
CH=CH-COOCH3
CH3-C-CH2-COOCH3/CH3ONa
H3OOC
1. KOH
2. HCl

(VII)

Die Cyclohexenonderivate der Formel Ia

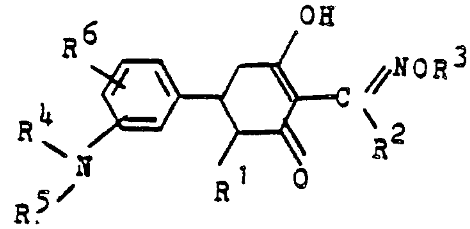

(Ia),

und die Tricarbonylverbindungen der Formel II

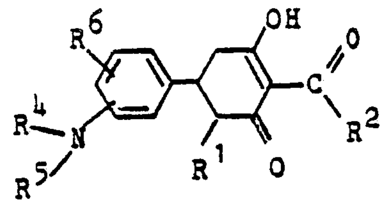

(II),

in denen

$R^1$ und $R^4$ Wasserstoff,

$R^2$ Alkyl mit 1 bis 4 C-Atomen,

$R^3$ Alkyl mit 1 bis 4 C-Atomen, Alkenylmit 3 oder 4 C-Atomen, Halogenalkenyl mit 3 oder 4 C-Atomen und 1 bis 3 Halogensubstituenten oder Propargyl,

$R^5$ aliphatisches Acyl mit 2 bis 8 C-Atomen, Formyl, Cycloalkylcarbonyl mit 4 bis 7 C-Atomen, Methoxyalkylcarbonyl, gegebenenfalls durch Nitro, Halogen, Alkyl, Alkoxy oder Haloalkyl substituiertes Benzoyl, und

$R^6$ Alkyl, Halogen, Hydroxy oder Alkoxy bedeuten,

können durch Umsetzung einer Verbindung der Formel Ia bzw. II, in der $R^5$ Wasserstoff bedeutet, mit elektrophilen Reagentien der Formel $R^5Y$ (III), in denen $R^5$ die oben angegebenen Bedeutungen hat und Y ein Austritts-gruppe, wie Chor, Brom oder Carboxylat ist, erhalten werden.

Die Umsetzung wird gegebenenfalls in einem inerten organische Lösungsmittel durchgeführt. Geeignet sind z.B. Kohlenwasserstoffe, wie Ligroin, Benzin, Toluol, Pentan, Cyclohexan, Halogenkohlenwasserstoffe, wie Methylenchlorid, Chloroform, Dichlorethan Chlorbenzol, o-, m- oder p-Dichlorbenzol, Nitrokohlenwasserstoffe, wie Nitrobenzol, Nitromethan, Nitrile, wie Acetonitril, Butyronitril, Benzonitril, Ether, wie Diethylether, Tetrahydrofuran, Dioxan, Ester, wie Essigsäureethylester, Propionsäuremethylester, Ketone, wie Aceton, Methylethylketon, oder Amide wie N,N-Dimethylformamid oder Formamid. Auch Gemsiche dieser Lösungsmittel können verwendet werden. Die Menge an Lösungsmittel, bezogen auf eingesetztes Anilinderivat beträgt 100 bis 5 000 Gew.-%.

Zweckmäßigerweise wird die Umsetzung in Gegenwart eines üblichen Säureakzeptors durchgeführt. In Betracht kommen Alkalihydroxide, Alkalicarbonate, Alkalihydrogencarbonate, Erdalkalihydroxide, Erdalkalicarbonate, Erdalkalihydrogencarbonate, Erdalkalioxide oder Amine, beispielsweise Natriumhydrogencarbonat, Kaliumcarbonat, Triethylamin, Tributylkamin, Pyridin, N,N-Dimethylanilin, N,N-Dimethyl-N-cyclohexylamin, Chinolin. Die Menge an Saüreakzeptor beträgt 1 bis 4 Mol, bezogen auf 1 Mol eingesetztes Anilinderivat.

Die Reaktionstemperatur liegt zwischen −20 und +150°C, verzugsweise zwischen 20 und 80°C. Die elektrophilen Reagentien $R^5Y$ und die als Ausgangsverbindungen verwendeten Verbindungen der Formeln Ia bzw. II werden bevorzugt in äquimolarem Verhältnis eingesetzt.

Beispiels für Verbindungen der Formel $R^5Y$ sind Carbonsäurehalogenide, Carbonsäureanhydride, Chlorameisensäureester, wie Carbonsäurehalogenide der Formel

$$R^8\text{—CO—Hal},$$

in der

$R^8$ für Alkyl mit 1 bis 7 C-Atomen, Cycloalkyl mit 3 bis 5 C-Atomen, Methoxyalkyl mit 2 bis 4 C-Atomen oder gegebenenfalls durch Nitro, Halogen, Alkyl mit 1 bis 4 C-Atomen, Alkoxy mit 1 bis 3 C-Atomen oder Haloalkyl substituiertes Phenyl steht, Formylacetanhydrid der Formel

$$\mathrm{H{-}\overset{\displaystyle O}{\overset{\|}{C}}{-}O{-}\overset{\displaystyle O}{\overset{\|}{C}}{-}CH_3},$$

Man erhält die benötigen Derivate Ia, in denen $R^5$ Wasserstoff bedeutet, durch katalytische Hydrierung der entsprechenden Nitro-Verbindungen der Formel V,

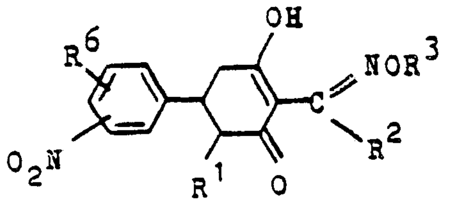

(V)

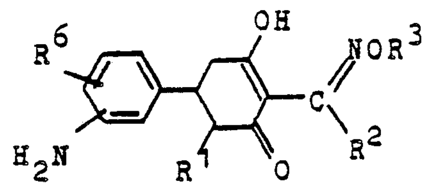

(Ib)

Hierbei wird gegebenenfalls in Gegenwart eines indifferenten Lösungsmittels wie z.B. Tetrahydrofuran, Dioxan, $C_1$ bis $C_4$-Alkohole, Ethylenglykolmonomethylether, Ethylenglykolmonoethylether, N,N-Dimethylformamid, Pyridin, Essigester, Aceton, Wasser oder Gemische dieser Lösungsmittel, unter einem Druck zwischen 1 bis 5 bar, bevorzugt Normaldruck, unter Zugabe eines überlichen Hydrierkatalysators wie Palladium, Platin, Nickel bei Temperaturen zwischen 0 bis 80°C, bevorzugt Raumtemperatur gearbeitet.

Die zur Herstellung der Verbindungen V erforderlichen Derivate der Formel VI können nach verschiedenen Methoden erhalten werden:

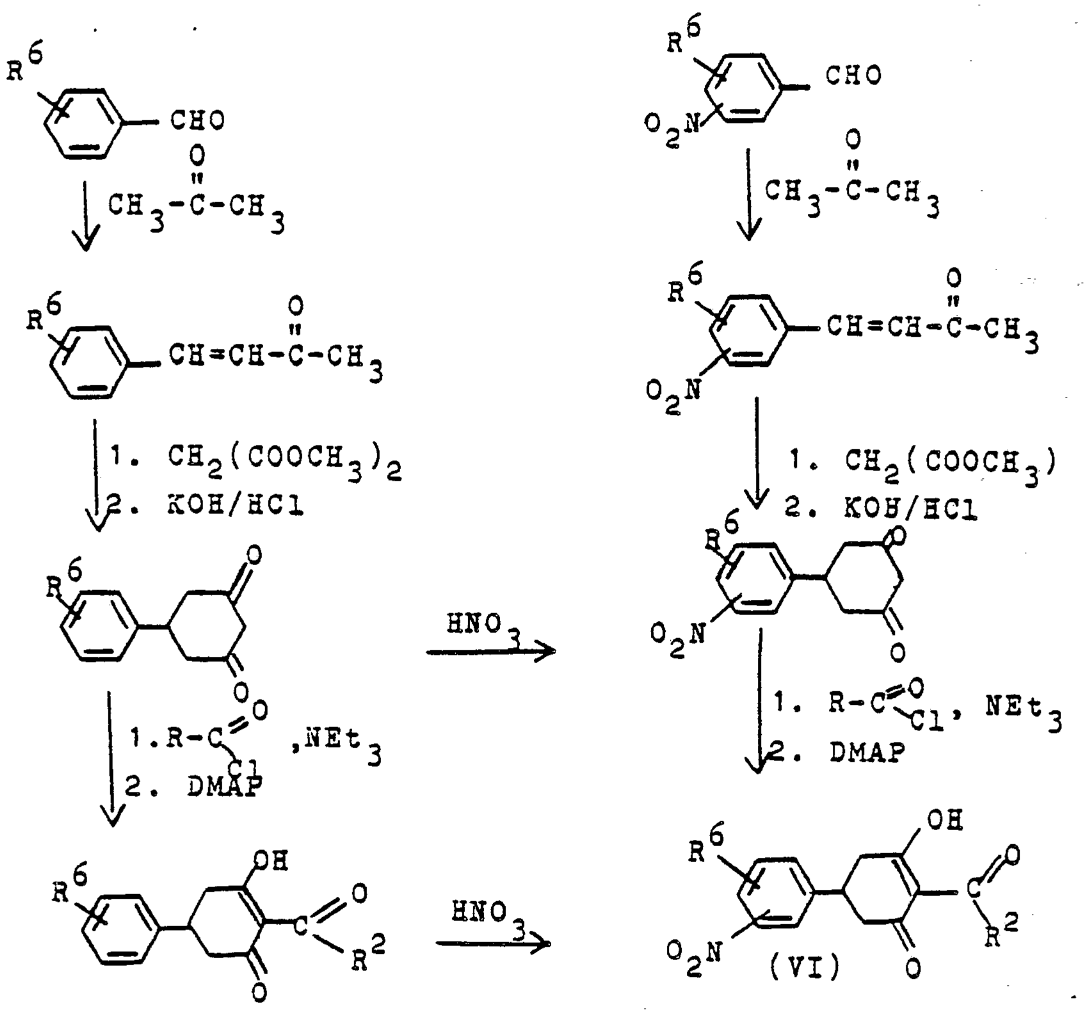

Dabei hängt Ort des Eintritts der Nitrogruppe auf "Dicarbonyl- oder Tricarbonyl-Stufe" nach den Regeln elektrophiler Aromatensubstitution in entscheidender Weise von $R^6$ ab, z.B.:

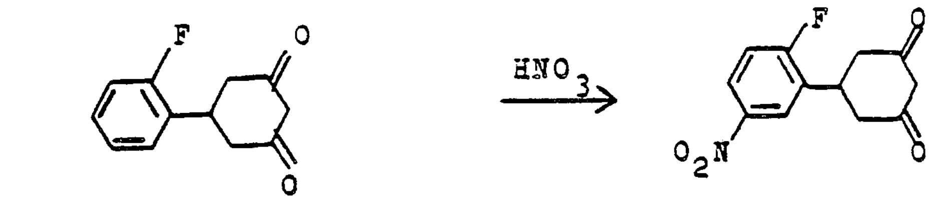


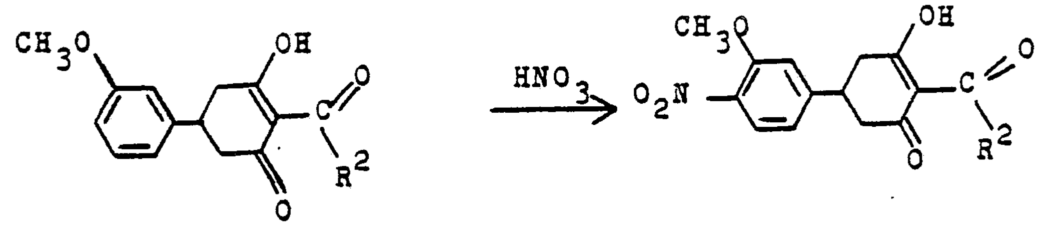


Die folgenden Beispiele erläutern die Herstellung der Cyclohexenonderivate der Formel I:

Beispiel 1

5,3 g 2-Butyryl-(3-nitro-4-methoxyphenyl)-cyclohexan-1,3-dion werden in 300 ml Tetrahydrofuran in Anwesenheit von 3 g Pd/Kohle (10%) drucklos bei 25°C hydriert. Nach Aufnahme von 0,9 l Wasserstoff wird über Natriumsulfat getrocknet, filtriert, mit 1,3 g Natriumhydrogencarbonat versetzt und mit 1,2 g Acetylchlorid zur Reaktion gebracht. Nach 16 stündigem Rühren wird filtriert und eingeengt. Das verbleibende Öl wird mit Diethylether zur Kristallisation gebracht und abgesaugt. Man erhält 3 g (55% Ausbeute) 5-(3-Acetylamino-4-methoxy-phenyl)-2-butyryl-3-hydroxy-cyclohex-2-en-1-on vom Fp. 102—106°C.

Beispiel 2

3 g 5-(3-Acetylamino-4-methoxy-phenyl)-2-butyryl-3-hydroxy-cyclohex-2-en-1-on werden zusammen mit 0,95 g O-Allylhydroxylaminhydrochlorid und 0,8 g Natriumhydrogencarbonat in 40 ml Methanol 16 Stunden gerührt. Nach dem Einengen wird mit Methylenchlorid versetzt, mit Wasser ausgewaschen, getrocknet und eingeengt. Der verbleibende Rückstand wird mit Diisopropylether verrührt und abgesaugt. Man erhält 2,0 g (57% Ausbeute) 5-(3-Acetylamino-4-methoxy-phenyl)-2-(allyloxyiminobutyl)-3-hydroxy-cyclohex-2-en-1-on vom Fp. 136—137°C (Verbindung Nr. 11).

Beispiel 6

31 g 2-Acetyl-3-hydroxy-5-(2-methoxyphenyl)-cyclohex-2-en-1-on werden in 140 ml Eisessig gelöst und bei 25°C unter Kühlung mit 140 ml konzentrierter Schwefelsäure versetzt, nach dem schnellen Zutropfen von 7,7 g 98 %iger Salpetersäure wird 30 Min. gerührt und dann in eine Eis/Methylenchlorid-Mischung eingegossen, die organische Phase wird abgetrennt, mit Wasser gewaschen und über Natriumsulfat getrocknet. Das nach dem Einengen erhaltene dunkle Öl wird in Methylenchlorid über Kieselgel filtriert und eingeengt. Man erhält 27,56 g (76% Ausbeute) gelbliche Kristalle, die zu 20% aus 2-Acetyl-3-hydroxy-5-(2-methoxy-3-nitrophenyl)-cyclohex-2-en-1-on (A) und zu 80% aus 2-Acetyl-3-hydroxy-5-(2-methoxy-5-nitrophenyl)-cyclohex-2-en-1-on (B) bestehen, Mischschmelzpunkt 145—151°C.

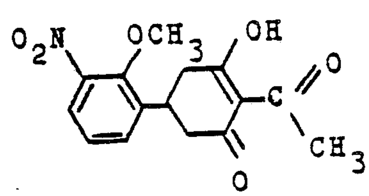

(A)

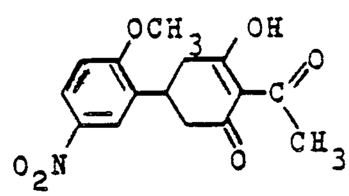

(B)

15 g des obigen Produktgemisches bestehend aus A und B (1:4) werden zusammen mit 4,2 g Natriumhydrogencarbonat und 4,6 g O-Ethylhydroxylammoniumchlorid in Methanol 16 Stunden gerührt, eingeengt, in Methylenchlorid aufgenommen, mit Wasser ausgeschüttelt, getrocknet und eingeent. Der Rückstand wird in Diisopropylether verrieben und abgesaugt. Man erhält 15,5 g (91% Ausbeute) eines wiederum 1:4 Gemisches der entsprechenden O-Ethyloximether C und D. Mischschmelzpunkt 132—142°C.

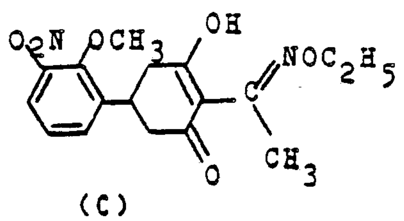

(C)

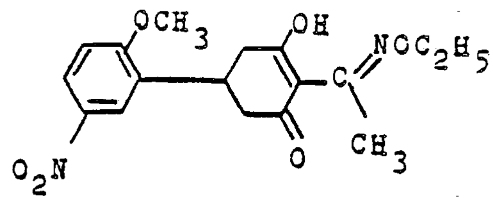

(D)

10,3 g des Produktgemisches C:D = 1:4 werden in 500 ml Tetrahydrofuran in Gegenwart von 3 g Palladium auf Kohle (10%) bei 25°C drucklos hydriert. Nach Aufnahme von 3 l Wasserstoff wird die Mischung über Natriumsulfat getrocknet, filtriert und eingeengt. Man erhält in quantitativer Ausbeute 9,5 g eines öligen 1:4 Gemisches der entsprechenden Aniline E und F.

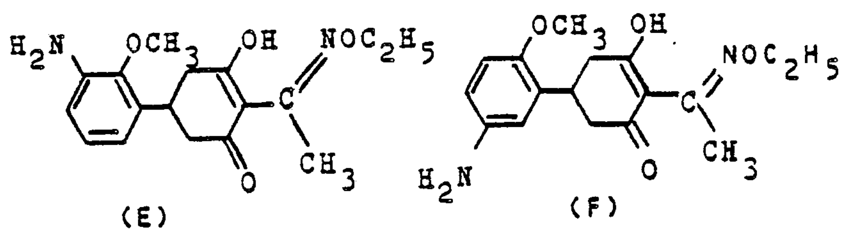

(E) (F)

9 g dieses Anilingemisches werden in 50 ml Tetrahydrofuran gelöst, mit 2,4 g Natriumhydrogencarbonat versetzt und unter Rühren mit 2,2 g Acetylchlorid zur Reaktion gebracht. Nach 16 stündigem Rühren wird abfiltriert, eingeengt und das verbleibende Öl mit Essigester über Kieselgel chromatographiert. Man erhält 0,9 g (9% Ausbeute) an isomerenreinem 5-(3-Acetamido-2-methoxyphenyl)-2-(1-ethoxyiminoethyl)-3-hydroxy-cyclohex-2-en-1-on,

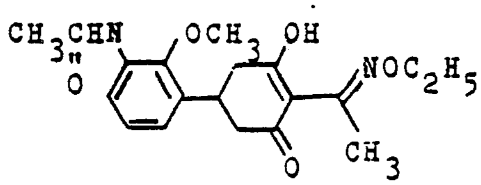

vom Fp. 124 - 126°C
(Verbindung Nr. 91)

und 3,6 g (35% Ausbeute) an isomerenreinem 5-(5-Acetamido-2-methoxyphenyl)-2-(1-ethoxyiminoethyl)-3-hydroxy-cyclohex-2-en-1-on.

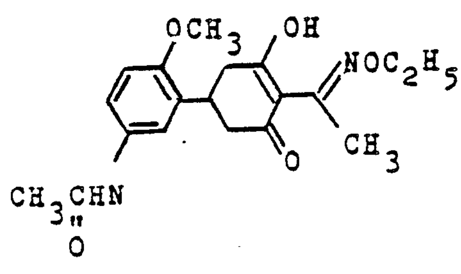

vom Fp. 152 - 153° C
(Verbindung Nr. 95)

Beispiel 7

60 g 5-(2-Fluorphenyl)-cyclohexan-1,3-dion werden bei −20°C in 500 ml konzentrierter Schwefelsäure gelöst und mit 18,3 g 98 %iger Salpetesäure bei −20°C innerhalb von 60 Minuten nitriert. Das Reaktionsgemisch wird in 5 l Eiswasser eingerührt. Die ausgefallenen Kristalle werden abgesaugt und getrocknet. Man erhält 60,5 g (83% Ausbeute) an 5-(2-Fluor-5-nitrophenyl)-cyclohexan-1,3-dion vom Fp. 171—173°C.

54 g 5-(2-Fluor-5-nitrophenyl)-cyclohexan-1,3-dion werden in 500 ml Methylenchlorid gelöst, mit 18,7 g Pyridin versetzt und bei RT innerhalb von 1 Stunde mit 25,2 g Buttersäurechlorid O-acyliert. Nach 16 stündigem Rühren wird mit Eiswasser, 5 %iger Natriumhydrogencarbonatlösung und nochmels mit Eiswasser ausgeschüttelt, über Natriumsulfat getrocknet und eingeengt. Das verbleibende Öl wird in etwa 300 ml Essigester aufgenommen und zusammen mit 3 g 4-Dimethylaminopyridin 5 Tage stehengelassen.

Die organische Phase wird 2mal mit 5 %iger Salzsäure ausgeschüttelt, getrocknet und eingeengt. Der verbleibende Rückstand wird aus Isopropanol umkristallisiert. Man erhält 33 g (48% Ausbeute) an 2-Butyryl-5-(2-fluor-5-nitrophenyl)-3-hydroxy-cyclohex-2-en-1-on vom Fp. 132°C.

25 g 2-Butyryl-5-(2-fluor-5-nitrophenyl)-3-hydroxy-cyclohex-2-en-1-on, 7,4 Natriumhydrogencarbonat und 8,6 g O-Ethylhydroxyammoniumchlorid werden 16 Stunden in 100 ml Methanol gerührt, eingeengt, in Methylenchlorid aufgenommen, mit Wasser gewaschen, getrocknet und wieder eingeengt. Man erhält 24 g (84% Ausbeute) 2-(1-Ethoxyiminobutyl)-5-(2-fluor-5-nitrophenyl)-3-hydroxy-cyclohex-2-en-1-on als viskoses Öl.

23 g dieses Öls werden in 500 ml Tetrahydrofuran in Anwesenheit von 5 g Palladium auf Kohle (10%) bei 25°C drucklos hydriert. Nach Aufnahme von 4,5 l Wasserstoff wird getrocknet, abfiltriert und eingeengt. Man erhält 19,6 g (93% Ausbeute) 5-(5-Amino-2-fluorphenyl)-2-(1-ethoxyiminobutyl)-3-hydroxy-cyclohex-2-en-1-on als viskoses Öl.

Entsprechend werden folgende Cyclohexenonderivats der Formel I erhalten:

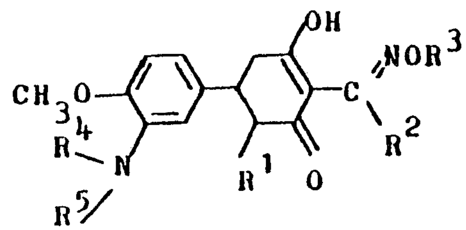

| Wirkstoff Nr. | $R^5$ | $R^1$ | $R^2$ | $R^3$ | $R^4$ | Fp. [°C] |
|---|---|---|---|---|---|---|
| 5 | Methyl | H | n-$C_3H_7$ | $C_2H_5$ | $CH_3$ | |
| 6 | Methyl | H | n-$C_3H_7$ | $CH_2$-CH=$CH_2$ | $CH_3$ | |
| 7 | Acetyl | H | n-$C_3H_7$ | $C_2H_5$ | H | 139 - 141 |
| 8 | Acetyl | $COOCH_3$ | $C_2H_5$ | $C_2H_5$ | H | 54 - 61 |
| 9 | Acetyl | H | $CH_3$ | $C_2H_5$ | H | 130 - 133 |
| 10 | Acetyl | H | $C_2H_5$ | $C_2H_5$ | H | 124 - 126 |
| 11 | Acetyl | H | $C_3H_7$ | $CH_2CH$=$CH_2$ | H | 136 - 137 |
| 12 | Butyryl | H | n-$C_3H_7$ | $C_2H_5$ | H | |
| 13 | Butyryl | H | n-$C_3H_7$ | $CH_2$-CH=$CH_2$ | H | |
| 14 | Butyryl | H | $C_2H_5$ | $CH_2$-CH=$CH_2$ | H | |

| Wirkstoff Nr. | $R^5$ | $R^1$ | $R^2$ | $R^3$ | $R^4$ | Fp. [°C] |
|---|---|---|---|---|---|---|
| 15 | Butyryl | H | $C_2H_5$ | $C_2H_5$ | H | |
| 16 | Formyl | H | $n-C_3H_7$ | $C_2H_5$ | H | 117 - 118 |
| 17 | Formyl | H | $n-C_3H_7$ | $CH_2-CH=CH_2$ | H | |
| 18 | Formyl | H | $C_2H_5$ | $CH_2-CH=CH_2$ | H | |
| 19 | Formyl | H | $C_2H_5$ | $C_2H_5$ | H | |
| 20 | Formyl | H | $n-C_3H_7$ | $CH_2-CH=CHCl$ | H | |
| 21 | Cyclopropylcarbonyl | H | $n-C_3H_7$ | $C_2H_5$ | H | 131 - 135 |
| 22 | Cyclopropylcarbonyl | H | $n-C_3H_7$ | $CH_2-CH=CH_2$ | H | |
| 23 | Cyclopropylcarbonyl | H | $C_2H_5$ | $CH_2-CH=CH_2$ | H | |
| 24 | Cyclopropylcarbonyl | H | $C_2H_5$ | $C_2H_5$ | H | |
| 25 | Methoxyacetyl | H | $n-C_3H_7$ | $C_2H_5$ | H | 138 - 140 |
| 26 | Methoxyacetyl | H | $n-C_3H_7$ | $CH_2-CH=CH_2$ | H | |
| 27 | Methoxyacetyl | H | $C_2H_5$ | $CH_2-CH=CH_2$ | H | |
| 28 | Methoxyacetyl | H | $C_2H_5$ | $C_2H_5$ | H | 127 - 130 |
| 29 | 3-Methoxypropionyl | H | $n-C_3H_7$ | $C_2H_5$ | H | 93 - 97 |
| 30 | 3-Methoxypropionyl | H | $n-C_3H_7$ | $CH_2-CH=CH_2$ | H | |
| 31 | 3-Methoxypropionyl | H | $C_2H_5$ | $CH_2-CH=CH_2$ | H | |
| 32 | 3-Methoxypropionyl | H | $C_2H_5$ | $C_2H_5$ | H | |
| 33 | Benzoyl | H | $n-C_3H_7$ | $C_2H_5$ | H | 110 - 112 |
| 34 | Benzoyl | H | $n-C_3H_7$ | $CH_2-CH=CH_2$ | H | |

| Wirkstoff Nr. | $R^5$ | $R^1$ | $R^2$ | $R^3$ | $R^4$ | Fp. [°C] |
|---|---|---|---|---|---|---|
| 35 | Benzoyl | H | $C_2H_5$ | $CH_2-CH=CH_2$ | H | |
| 36 | Benzoyl | $COOCH_3$ | $C_2H_5$ | $C_2H_5$ | H | |
| 37 | 3-Nitrobenzoyl | H | $n-C_3H_7$ | $C_2H_5$ | H | |
| 38 | 3-Methoxybenzoyl | H | $n-C_3H_7$ | $C_2H_5$ | H | |
| 39 | 4-Chlorbenzoyl | H | $C_2H_5$ | $C_2H_5$ | H | |
| 138 | Methoxyacetyl | H | $CH_3$ | $C_2H_5$ | H | 120 - 124 |
| 139 | Benzoyl | H | $C_2H_5$ | $C_2H_5$ | H | 120 - 121 |
| 140 | Benzoyl | H | $CH_3$ | $C_2H_5$ | H | 134 - 139 |

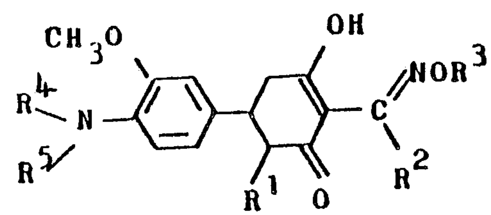

| Wirkstoff Nr. | $R^5$ | $R^1$ | $R^2$ | $R^3$ | $R^4$ | Fp. [°C] |
|---|---|---|---|---|---|---|
| 81 | Acetyl | H | $n-C_3H_7$ | $C_2H_5$ | H | |
| 82 | Acetyl | H | $n-C_3H_7$ | $CH_2-CH=CH_2$ | H | |
| 143 | Acetly | H | $n-C_3H_7$ | $n-C_3H_7$ | H | 69 - 72 |
| 144 | Benzoyl | H | $n-C_3H_7$ | $C_2H_5$ | H | 112 - 114 |
| 145 | Methoxyacetyl | H | $n-C_3H_7$ | $C_2H_5$ | H | 95 - 99 |

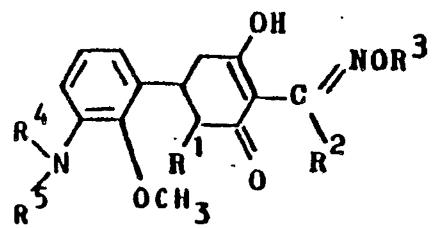

| Wirkstoff Nr. | $R^5$ | $R^1$ | $R^2$ | $R^3$ | $R^4$ | Fp. [°C] |
|---|---|---|---|---|---|---|
| 91 | Acetyl | H | $CH_3$ | $C_2H_5$ | H | 124 - 126 |
| 146 | Acetyl | H | $C_2H_3$ | $C_2H_5$ | H | |
| 147 | Acetyl | H | $C_2H_5$ | $CH_2CH_2Cl$ | H | |
| 148 | Acetyl | H | $C_2H_5$ | (E)-$CH_2$-CH=CH-$CH_3$ | H | |
| 149 | Acetyl | H | $C_2H_5$ | (E)-$CH_2$-CH=CH-$CH_3$ | H | |
| 150 | Acetyl | H | $C_2H_5$ | $CH_2$-CH=$CH_2$ | H | |
| 151 | Acetyl | H | n-$C_3H_7$ | $C_2H_5$ | H | 96 - 98 |
| 152 | Acetyl | H | n-$C_3H_7$ | $CH_2$-$CH_2Cl$ | H | |
| 153 | Acetyl | H | n-$C_3H_7$ | $CH_2CH_2F$ | H | |
| 154 | Acetyl | H | n-$C_3H_7$ | (E)-$CH_2$-CH=CH-$CH_3$ | H | |
| 155 | Acetyl | H | n-$C_3H_7$ | (E)-$CH_2$-CH=CH-Cl | H | |
| 156 | Acetyl | H | n-$C_3H_7$ | $CH_2$-CH=$CH_2$ | H | |

| Wirkstoff Nr. | $R^5$ | $R^1$ | $R^2$ | $R^3$ | $R^4$ | Fp. [°C] |
|---|---|---|---|---|---|---|
| 157 | Propionyl | H | $C_2H_5$ | $C_2H_5$ | H | |
| 158 | Propionyl | H | $C_2H_5$ | $CH_2-CH=CH_2$ | H | |
| 159 | Propionyl | H | $n-C_3H_7$ | $CH_2-CH=CH_2$ | H | |
| 160 | Propionyl | H | $n-C_3H_7$ | $C_2H_5$ | H | |
| 161 | Butyryl | H | $C_2H_5$ | $C_2H_5$ | H | |
| 162 | Butyryl | H | $C_2H_5$ | $CH_2-CH=CH_2$ | H | |
| 163 | Butyryl | H | $n-C_3H_7$ | $CH_2-CH=CH_2$ | H | |
| 164 | Butyryl | H | $n-C_3H_7$ | $C_5H_5$ | H | |
| 169 | Methoxyacetyl | H | $C_2H_5$ | $C_2H_5$ | H | |
| 170 | Methoxyacetyl | H | $C_2H_5$ | $CH_2-CH=CH_2$ | H | |
| 171 | Methoxyacetyl | H | $n-C_3H_7$ | $CH_2-CH=CH_2$ | H | |
| 172 | Methoxyacetyl | H | $n-C_3H_7$ | $C_2H_5$ | H | |
| 173 | Benzoyl | H | $n-C_3H_7$ | $C_2H_5$ | H | |
| 174 | Benzoyl | H | $C_2H_5$ | $C_2H_5$ | H | |
| 175 | Benzoyl | H | $C_2H_5$ | $CH_2-CH=CH_2$ | H | |
| 176 | Benzoyl | H | $n-C_3H_7$ | $CH_2-CH=CH_2$ | H | |

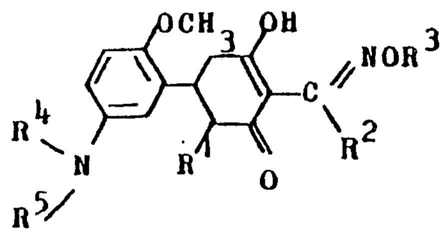

| Wirkstoff Nr. | $R^5$ | $R^1$ | $R^2$ | $R^3$ | $R^4$ | Fp. [°C] |
|---|---|---|---|---|---|---|
| 95 | Acetyl | H | $CH_3$ | $C_2H_5$ | H | 152 - 153 |
| 96 | Acetyl | H | $C_2H_5$ | $C_2H_5$ | H | 133 - 135 |
| 97 | Acetyl | H | $n\text{-}C_3H_7$ | $C_2H_5$ | H | 138 - 140 |
| 177 | 3-Methoxypropionyl | H | $n\text{-}C_3H_7$ | $C_2H_5$ | H | (NMR-Daten) |
| 178 | 3-Methoxypropionyl | H | $C_2H_5$ | $C_2H_5$ | H | |
| 179 | Benzoyl | H | $C_2H_5$ | $C_2H_5$ | H | 179 - 180 |
| 180 | Benzoyl | H | $n\text{-}C_3H_7$ | $C_2H_5$ | H | 134 - 136 |

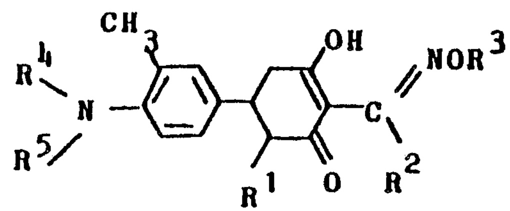

| Wirkstoff Nr. | $R^5$ | $R^1$ | $R^2$ | $R^3$ | $R^4$ | Fp. [°C] |
|---|---|---|---|---|---|---|
| 109 | Acetyl | H | $n-C_3H_7$ | $C_2H_5$ | H | 119 |
| 110 | Acetyl | H | $n-C_3H_7$ | $CH_2-CH=CH_2$ | H | |
| 195 | Methoxyacetyl | H | $n-C_3H_7$ | $C_2H_5$ | H | (NMR-Daten) |
| 196 | Methoxyacetyl | H | $n-C_3H_7$ | $CH_2-CH=CH_2$ | H | |
| 197 | Methoxyacetyl | H | $C_2H_5$ | $CH_2-CH=CH_2$ | H | |
| 198 | Methoxyacetyl | H | $C_2H_5$ | $C_2H_5$ | H | 158 - 160 |
| 213 | Benzoyl | H | $n-C_3H_7$ | $C_2H_5$ | H | 145 - 148 |
| 214 | Benzoyl | H | $C_2H_5$ | $C_2H_5$ | H | 123 - 126 |
| 217 | Acetyl | H | $C_2H_5$ | $C_2H_5$ | H | 153 - 156 |

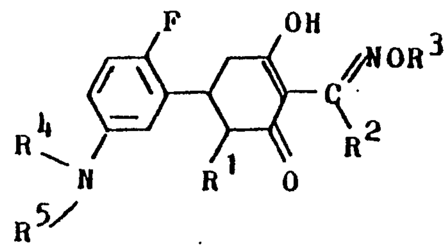

| Wirkstoff Nr. | $R^5$ | $R^1$ | $R^2$ | $R^3$ | $R^4$ | Fp. [°C] |
|---|---|---|---|---|---|---|
| 114 | Acetyl | H | n-$C_3H_7$ | $C_2H_5$ | H | 120 - 122 |
| 115 | Benzoyl | H | n-$C_3H_7$ | $C_2H_5$ | H | 116 - 117 |

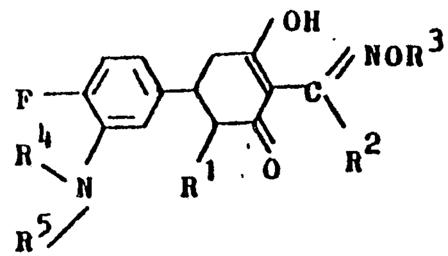

| Wirkstoff Nr. | $R^5$ | $R^1$ | $R^2$ | $R^3$ | $R^4$ | Fp. [°C] |
|---|---|---|---|---|---|---|
| 117 | Acetyl | H | $n-C_3H_7$ | $C_2H_5$ | H | 120 - 122 |
| 223 | Acetyl | H | $C_2H_5$ | $C_2H_5$ | H | |
| 224 | Acetyl | H | $n-C_3H_7$ | $CH_2-CH=CH_2$ | H | 109 - 111 |
| 225 | Acetyl | H | $C_2H_5$ | $CH_2CH_2Cl$ | H | |
| 226 | Acetyl | H | $n-C_3H_7$ | (E)-$CH_2-CH=CH-CH_3$ | H | 121 - 123 |
| 227 | Acetyl | H | $C_2H_5$ | (E)-$CH_2-CH=CH-Cl$ | H | |
| 228 | Methoxyacetyl | H | $n-C_3H_7$ | $C_2H_5$ | H | 94 - 96 |
| 229 | Methoxyacetyl | H | $n-C_3H_7$ | $CH_2-CH=CH_2$ | H | |
| 230 | Methoxyacetyl | H | $C_2H_5$ | $C_2H_5$ | H | |
| 231 | Methoxyacetyl | H | $C_2H_5$ | $CH_2-CH=CH_2$ | H | |
| 232 | Acetyl | H | $n-C_3H_7$ | (E)-$CH_2-CH=CH-Cl$ | H | 128 - 130 |

| Wirkstoff Nr. | $R^5$ | $R^1$ | $R^2$ | $R^3$ | $R^4$ | Fp. [°C] |
|---|---|---|---|---|---|---|
| 233 | Benzoyl | H | $C_2H_5$ | $C_2H_5$ | H | |
| 234 | Benzoyl | H | $C_2H_5$ | $CH_2-CH=CH_2$ | H | |
| 235 | Benzoyl | H | $n-C_3H_7$ | $CH_2-CH=CH_2$ | H | |
| 236 | Benzoyl | H | $n-C_3H_7$ | $C_2H_5$ | H | 125- 128 |
| 237 | 3-Methoxypropionyl | H | $n-C_3H_7$ | $C_2H_5$ | H | 112 - 114 |
| 238 | 3-Methoxypropionyl | H | $C_2H_5$ | $C_2H_5$ | H | |
| 251 | Propionyl | H | $n-C_3H_7$ | $C_2H_5$ | H | |
| 252 | Propionyl | H | $C_2H_5$ | $C_2H_5$ | H | |
| 253 | Butyryl | H | $n-C_3H_7$ | $C_2H_5$ | H | |
| 254 | Butyryl | H | $C_2H_5$ | $C_2H_5$ | H | |
| 255 | Cyclohexylcarbonyl | H | $n-C_3H_7$ | $C_2H_5$ | H | |
| 256 | Cyclohexylcarbonyl | H | $C_2H_5$ | $C_2H_5$ | H | |

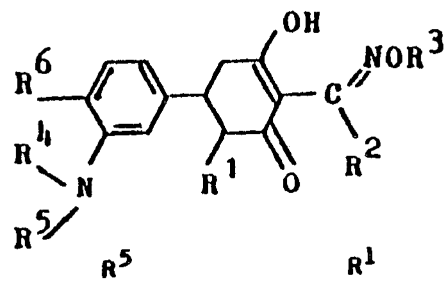

| Wirkstoff Nr. | $R^6$ | $R^5$ | $R^1$ | $R^2$ | $R^3$ | $R^4$ | Fp. [°C] |
|---|---|---|---|---|---|---|---|
| 121 | Chlor | Acetyl | H | $n-C_3H_7$ | $C_2H_5$ | H | 136 - 139 |
| 122 | Hydroxy | Acetyl | H | $n-C_3H_7$ | $C_2H_5$ | H | 125 - 126 |
| 123 | tert.-Butoxy | Acetyl | H | $n-C_3H_7$ | $C_2H_5$ | H | |
| 262 | Hydroxy | Acetyl | H | $C_2H_5$ | $C_2H_5$ | H | 144 - 146 |
| 264 | Hydroxy | Benzoyl | H | $n-C_3H_7$ | $C_2H_5$ | H | 150 - 152 |
| 265 | Hydroxy | Benzoyl | H | $C_2H_5$ | $C_2H_5$ | H | 122 - 126 |
| 268 | Chlor | Acetyl | H | $n-C_3H_7$ | (E)-$CH_2-CH=CH-Cl$ | H | 78 - 80 |
| 269 | Chlor | Benzoyl | H | $n-C_3H_7$ | $C_2H_5$ | H | 124 - 127 |
| 270 | Chlor | Methoxyacetyl | H | $n-C_3H_7$ | $C_2H_5$ | H | 98 - 103 |

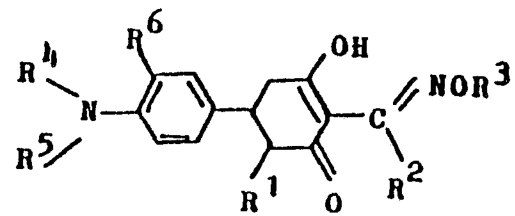

| Wirkstoff Nr. | $R^6$ | $R^5$ | $R^1$ | $R^2$ | $R^3$ | $R^4$ | Fp. [°C] |
|---|---|---|---|---|---|---|---|
| 276 | Chlor | Benzoyl | H | $n-C_3H_7$ | $C_2H_5$ | H | |
| 277 | Chlor | Benzoyl | H | $n-C_3H_7$ | $CH_2-CH=CH_2$ | H | |
| 278 | Chlor | Acetyl | H | $n-C_3H_7$ | $C_2H_5$ | H | |
| 279 | Chlor | Acetyl | H | $n-C_3H_7$ | $CH_2CH=CH_2$ | H | |
| 280 | Brom | Acetyl | H | $n-C_3H_7$ | $C_2H_5$ | H | |
| 281 | Bromo | Acetyl | H | $n-C_3H_7$ | $CH_2-CH=CH_2$ | H | |
| 282 | Brom | Benzoyl | H | $n-C_3H_7$ | $C_2H_5$ | H | |
| 283 | Brom | Benzoyl | H | $n-C_3H_7$ | $CH_2-CH=CH_2$ | H | |
| 284 | Fluor | Benzoyl | H | $C_2H_5$ | $C_2H_5$ | H | 151 - 153 |
| 285 | Fluor | Benzoyl | H | $C_2H_5$ | $C_2H_5$ | H | |
| 286 | Fluor | Benzoyl | H | $C_2H_5$ | $CH_2-CH=CH_2$ | H | |

| Wirkstoff Nr. | $R^6$ | $R^5$ | $R^1$ | $R^2$ | $R^3$ | $R^4$ | Fp. [°C] |
|---|---|---|---|---|---|---|---|
| 287 | Fluor | Benzoyl | H | $C_2H_5$ | (E)-$CH_2$-CH=CH-Cl | H | 156 - 157 |
| 288 | Fluor | Benzoyl | H | $C_2H_5$ | (E)-$CH_2$-CH=CH-$CH_3$ | H | |
| 289 | Fluor | Benzoyl | H | n-$C_3H_7$ | $C_2H_5$ | H | 163 - 165 |
| 290 | Fluor | Benzoyl | H | n-$C_3H_7$ | $CH_2CH_2Cl$ | H | |
| 291 | Fluor | Benzoyl | H | n-$C_3H_7$ | $CH_2$-CH=$CH_2$ | H | 121 - 123 |
| 292 | Fluor | Benzoyl | H | n-$C_3H_7$ | (E)-$CH_2$-CH=CH-Cl | H | 108 - 112 |
| 293 | Fluor | Benzoyl | H | n-$C_3H_7$ | (E)-$CH_2$-CH=CH-$CH_3$ | H | |
| 294 | Fluor | o-Toluyl | H | n-$C_3H_7$ | $C_2H_5$ | H | |
| 295 | Fluor | o-Toluyl | H | n-$C_3H_7$ | $CH_2$-CH=$CH_2$ | H | |
| 296 | Fluor | o-Toluyl | H | $C_2H_5$ | $C_2H_5$ | H | |
| 297 | Fluor | o-Toluyl | H | $C_2H_5$ | $CH_2$-CH=$CH_2$ | H | |
| 298 | Fluor | Acetyl | H | $C_2H_5$ | $C_2H_5$ | H | |
| 299 | Fluor | Acetyl | H | $C_2H_5$ | $CH_2$-CH=$CH_2$ | H | |
| 300 | Fluor | Acetyl | H | n-$C_3H_7$ | $C_2H_5$ | H | |
| 301 | Fluor | Acetyl | H | n-$C_3H_7$ | $CH_2$-CH=$CH_2$ | H | |

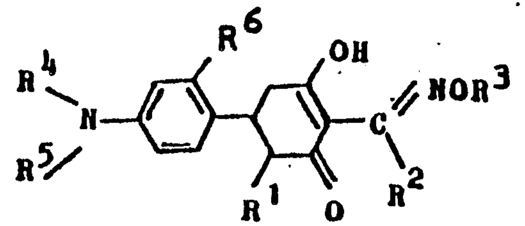

| Wirkstoff Nr. | $R^6$ | $R^5$ | $R^1$ | $R^2$ | $R^3$ | $R^4$ | Fp. [°C] |
|---|---|---|---|---|---|---|---|
| 303 | Fluor | Benzoyl | H | $C_2H_5$ | $C_2H_5$ | H | |
| 304 | Fluor | Benzoyl | H | $C_2H_5$ | $CH_2CH_2Cl$ | H | |
| 305 | Fluor | Benzoyl | H | $C_2H_5$ | $CH_2-CH=CH_2$ | H | |
| 306 | Fluor | Benzoyl | H | $C_2H_5$ | (E)-$CH_2-CH=CH-Cl$ | H | |
| 307 | Fluor | Benzoyl | H | $C_2H_5$ | (E)-$CH_2-CH=CH-CH_3$ | H | |
| 308 | Fluor | Benzoyl | H | n-$C_3H_7$ | $C_2H_5$ | H | (NMR-Daten) |
| 309 | Fluor | Benzoyl | H | n-$C_3H_7$ | $CH_2CH_2Cl$ | H | |
| 310 | Fluor | Benzoyl | H | n-$C_3H_7$ | $CH_2-CH=CH_2$ | H | |
| 311 | Fluor | Benzoyl | H | n-$C_3H_7$ | (E)-$CH_2-CH=CH-Cl$ | H | |
| 312 | Fluor | Benzoyl | H | n-$C_3H_7$ | (E)-$CH_2-CH=CH-CH_3$ | H | |
| 313 | Chlor | Benzoyl | H | $C_2H_5$ | $C_2H_5$ | H | |
| 314 | Chlor | Benzoyl | H | $C_2H_5$ | $CH_2-CH=CH_2$ | H | |
| 315 | Chlor | Benzoyl | H | n-$C_3H_7$ | $C_2H_5$ | H | |
| 316 | Chlor | Benzoyl | H | n-$C_3H_7$ | $CH_2-CH=CH_2$ | H | |

Die Cyclohexenonderivate der Formel I lassen sich durch charakteristische $^1H$-NMR spektroskopische Daten (chemische Verschiebung in δ-Werten (ppm) in $CDCl_3$, bezogen auf Tetramethylsilan als internen Standard; s = Singulett, d = Dublett, q = Quartett, m = Multiplett) kennzeichnen:

| Verbindung Nr. | Charakteristische Signale |
|---|---|
| 91 | 2,2 (s, 3H); 2,45 (s, 3H); 3,75 (s, 3H); 6.95 (d, 1H); 7.15 (t, 1H); 8,20 (d, 1H) |
| 95 | 2,2 (s, 3H); 2,5 (s, 3H); 3,8 (s, 3H); 6,8 (d, 1H); 7,45 (d, 1H) |
| 177 | 2,95 (t); 6,8 (d); 7,25 (m); 7,45 (m), 8,35 (s) |
| 195 | 2,15 (s); 3,4 (s); 8,1 (s); 6,9—7,1 (m); 7,85 (d) |
| 308 | 2,97 (t); 4,12 (q); 7,86 (d). |

Die Cyclohexenonderivate der Formel I und ihre Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich ganz nach den Verwendungszwecken; sie sollten in jedem Fall möglichst die feinste Verteilung der erfindungsgemäßen Wirkstoffe gewährleisten.

Zer Herstellung von direkt versprühbaren Lösungen, Emulsionen, Pasten oder Öldispersionen kommen Mineralölfraktionen von mittleren Naphthalinen, aromatische Kohlenwasserstoffe (Toluol, Xylol), Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflachenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

II. 20 Gewichtsteile der Verbindung Nr. 7 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 109 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000

Gewichtsteilen Wasser erhält man eine wäßrige Dispersion, die 0,02 Gewichtsprozent des Wirkstoffs enthält.

V. 20 Gewichtsteile des Wirkstoffs Nr. 7 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile des Wirkstoffs Nr. 33 werden mit 97 Gewichtsteilen feinteiligem Kaolin vermischt. Man erhält auf diese Weise ein Stäubemittel, das 3 Gewichtsprozent des Wirkstoffs enthält.

IX. 10 Gewichtsteile des Wirkstoffs Nr. 289 werden mit 90 Gewichtsteilen einer Mischung aus 93 Gewichtsteilen Xylol und 7 Gewichtsteilen des Anlagerungsproduktes von 8 Mol Ethylenoxid an 1 Mol Nonylphenol gemischt. Man erhält auf diese Weise eine Lösung, die 10 Gewichtsprozent des Wirkstoffs enthalt.

Die Applikation kann im Vorauflauf- oder im Nachauflaufverfahren erfolgen. Sind die Wirkstoffe bei Nachauflaufanwendung für gewisse Kulturpflanzen weniger verträglich, so können auch Aufbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,025 bis 3 kg/ha, vorzugsweise 0,06 bis 0,5 kg/ha.

Die Wirkung der Cyclohexenonderivate der Formel I auf das Pflanzenwachstum läßt sich durch Gewächshausversuche zeigen:

Als Kulturpflanzen dienen Plastikblumentöpfe mit 300 $cm^3$ Inhalt und lehmigem Sand mit etwa 1,5% Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelte sie danach. Die Sojapflanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmenge für die Nachauflaufbehandlung beträgt 0,06 bis 0,5 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Alopecurus myosuroides (Ackerfuchsschwanz), Avena fatua (Flughafer), Avena sativa (Hafer), Echinochloa crus-galli (Hühnerhirse), Glycine max (Sojabohnen), Lolium multiflorum (Ital. Raygras), Oryza sativa (Reis), Setaria italica (Kolbenhirse), Sinapis alba (Weißer Senf), Sorghum halepense (Sudangras, Wilde Mohrenhirse).

Bei Vorauflaufanwendung erweisen sich beispielsweise die Verbindungen Nr. 7 und 33 als herbizid wirksam gegen Pflanzen aus der Familie der Gräser, während Sinapis alba als ein Vertreter dikotyler Pflanzen völlig ungeschädigt bleibt.

Bei Nachauflaufbehandlung zeigen beispielsweise die Verbindungen Nr. 114 und 109 mit 0,5 bzw. 0,06 kg Wirkstoff/ha eine starke herbizide Aktivität gegen beispielhaft ausgwählte Gräser während Sojabohnen als dikotyle kulturpflanzen nicht geschädigt werden. Verbindung Nr. 7 eignet sich beispielsweise zur Bekämpfung von Hühnerhirse in Reis.

Schon mit geringen Aufwandmengen können z.B. mit Verbindung Nr. 109 unerwünschte Gräser bekämpft werden, Soja als dikotyle Beispielkultur bleibt unbeeinflußt.

Ebenso eignen sich beispielsweise die Verbindungen Nr. 16 und 91 zur Bekämpfung eines breiten Spektrums von unerwünschten grasartigen Pflanzen, wobei Soja als Kulturpflanze keinen Schaden erleidet.

Mit der beispielshaft ausgewählten Verbindung Nr. 117 lassen sich unerwünschte grasartige Pflanzen gut bekämpfen. Luzerne als Kulturpflanze erfährt dabei keine Schädigung.

Zur Bekämpfung wichtiger Ungräser in Reis eignet sich z.B. Verbindung Nr. 289. Sie verursacht dabei nur geringe Schäden an der Kulturpflanze.

In Anbetracht des erfaßbaren Wirkungsspektrums zur Unkrautbekämpfung, der Verträglichkeit für

Kulturpflanzen oder der unerwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glyoine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |

| Botanischer Name | Deutscher Name |
|---|---|
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolben-hirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |

| Botanischer Name | Deutscher Name |
| --- | --- |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenonderivate der Formel I und ihre Salze mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäurederivate und andere herbizide Wirkstoffe in Betracht.

Außerdem kann es von Nutzen sein, die Verbindungen der Formel I allein oder in Kombination mit anderen Herbiziden, auch noch mit weiteren Pflanzenschutzmitteln gemischt gemeinsam auszubringen, beispielsweise mit Mitteln zur Bekämpfung von Schädlingen oder phytopathogenen Pilsen bzw. Bakterien. Von Interesse ist ferner die Mischbarkeit mit Mineralsalzlösungen, welche zur Bekämpfung von Ernährungs- und Spurenelementmängeln eingesetzt werden. Es können auch nicht-phytotoxische Öle und Ölkonzentrate zugesetzt werden.

**Patentansprüche**

1. Cyclohexenonderivate der Formel I

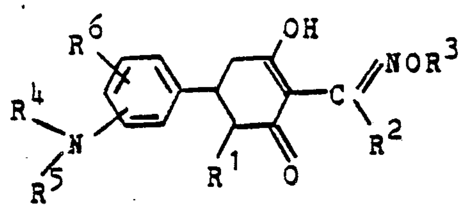

(I),

in der die Substituenten folgende Bedeutung haben:

$R^1$ Wasserstoff, Methoxycarbonyl, Ethoxycarbonyl oder Methyl,

$R^2$ Alkyl mit 1 bis 4 C-Atomen,

$R^3$ Alkyl mit 1 bis 4 C-Atomen, Alkenyl mit 3 oder 4 C-Atomen, Halogenalkenyl mit 3 oder 4 C-Atomen und 1 bis 3 Halogensubstituenten oder Propargyl,

$R^4$ Wasserstoff oder Alkyl mit 1 bis 4 C-Atomen,

$R^5$ aliphatisches Acyl mit 2 bis 8 C-Atomen, Formyl, Cycloalkylcarbonyl mit 4 bis 7 C-Atomen, Methoxyalkylcarbonyl, unsubstituiertes oder durch Nitro, Halogen, Alkyl, Alkoxy oder Haloalkyl substituiertes Benzoyl und

$R^6$ Alkyl, Halogen, Hydroxy oder Alkoxy,

und Salze dieser Verbindungen.

2. Cyclohexenonderivate der Formel I gemäß Anspruch 1, dadurch gekennzeichnet, daß $R^1$ Wasserstoff bedeutet.

3. Cyclohexenonderivate der Formel I gemäß Anspruch 1, dadurch gekennzeichnet, daß $R^1$ und $R^4$ Wasserstoff, $R^5$ Acetyl oder Benzoyl und $R^6$ Fluor oder Chlor bedeuten.

4. Verfahren zur Herstellung von Cyclohexenonderivaten der Formel I gemäß Anspruch 1, dadurch gekennzeichnet, daß man eine Tricarbonylverbindung der Formel II

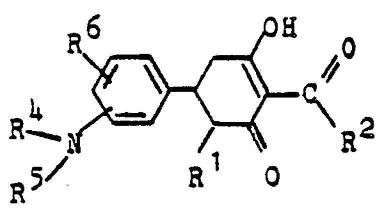

(II),

in der $R^1$, $R^2$, $R^4$, $R^5$ und $R^6$ die im Anspruch 1 genannten Bedeutungen haben,

a) mit Ammoniumverbindungen der Formel $R^3O—NH_3Y$, in der $R^3$ die in Anspruch 1 genannten Bedeutungen hat und Y ein Anion bedeutet, in einem inerten Verdünnungsmittel gegebenenfalls in Gegenwart von Wasser bei einer Temperatur zwischen 0 und 80°C in Gegenwart einer Base oder

b) mit gegebenenfalls in wäßriger Lösung vorliegenden Hydroxylaminen der Formel $R^3O—NH_2$, in der $R^3$ die im Anspruch 1 genannten Bedeutungen hat, in einem inerten Lösungsmittel umsetzt.

5. Verfahren zur Herstellung von Cyclohexenonderivaten der Formel Ia

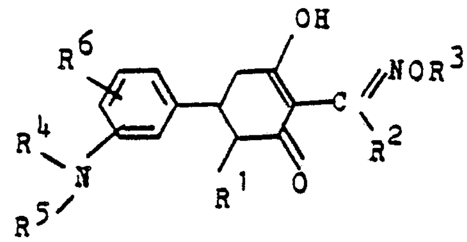

(Ia),

in der

$R^1$ und $R^4$ Wasserstoff und

$R^5$ aliphatisches Acyl mit 2 bis 8 C-Atomen, Formyl, Cycloalkylcarbonyl mit 4 bis 7 C-Atomen, Methoxyalkylcarbonyl oder gegebenenfalls durch Nitro, Halogen, Alkyl, Alkoxy oder Haloalkyl substituiertes Benzoyl bedeuten

und $R^2$, $R^3$ und $R^6$ in Anspruch 1 für Formel I genannen Bedeutungen haben, dadurch gekennzeichnet, daß man ein Cyclohexenonderivat der Formel I, in der $R^5$ Wasserstoff bedeutet und $R^1$, $R^2$, $R^3$, $R^4$ und $R^6$ die obengenannten Bedeutungen haben, gegebenenfalls in einem auf einen bestimmten pH-Wert gepufferten System, gegebenenfalls in Gegenwart eines säurebindenden Mittels bei einer Temperatur zwischen −20 und +150°C

mit einer Verbindung der Formel III

$$R^5Y \qquad (III),$$

in der $R^5$ die obengenannten Bedeutungen hat und Y eine Austrittsgruppe ist, umsetzt.

6. Verfahren zur Herstellung von Cyclohexenonderivaten der Formel Ib

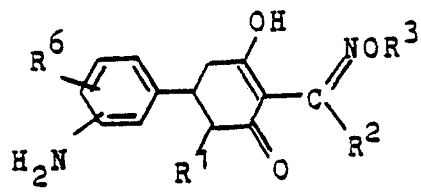

(Ib)

in der $R^1$, $R^2$ und $R^6$ die im Anspruch 1 angegebenen Bedeutungen haben und $R^3$ Alkyl mit 1 bis 4 C-Atomen bedeutet, dadurch gekennzeichnet, daß man eine Verbindung der Formel V

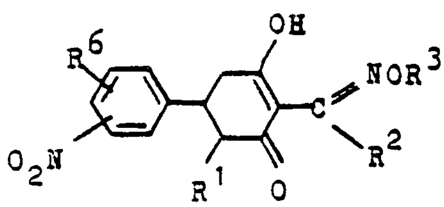

(V)

in der $R^1$, $R^2$, $R^3$ und $R^6$ die obengenannten Bedeutungen haben, mit Wasserstoff, gegebenenfalls unter Druck in Gegenwart eines inerten Lösungsmittels, gegebenenfalls unter Zusatz von Basen und in Gegenwart eines Hydrierkatalysators bei Temperaturen zwischen 0 und 80°C in an sich üblicher Weise zur Aminoverbindung reduziert.

7. Verfahren zur Herstellung von Tricarbonylverbindungen der Formel IIa

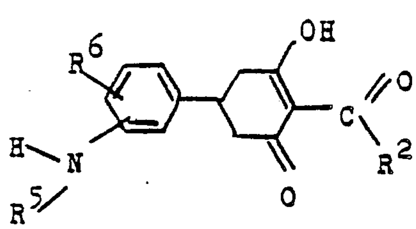

(IIa),

in der $R^2$, $R^5$ und $R^6$ die im Anspruch 1 genannten Bedeutungen haben, dadurch gekennzeichnet, daß man eine Verbindung der Formel VI

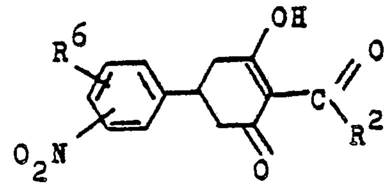

(VI),

in der $R^2$ und $R^6$ die oben angegebenen bedeutungen haben, in Gegenwart eines Hydrierkatalysators bei Temperaturen zwischen 0 und 80°C in an sich üblicher Weise zur Aminoverbindung reduziert und diese ohne weitere Isolierung mit den Verbindungen der Formel III und IV umsetzt.

8. Herbizid, enthaltend ein Cyclohexenonderivat der Formel I gemäß Anspruch 1.

9. Herbizid, enthaltend inerte Zusatzstoffe und ein Cyclohexenonderivat der Formel I gemäß Anspruch 1.

10. Herbizid nach Anspruch 9, dadurch gekennzeichnet, daß es 0,1 bis 95 Gew.% des Cyclohexenonderivats der Formel I enthält.

11. Herbizid nach Anspruch 9, dadurch gekennzeichnet, daß es ein Cyclohexenonderivats der Formel I enthält, wobei $R^1$ und $R^4$ Wasserstoff, $R^5$ Acetyl oder Benzoyl und $R^6$ Fluor oder Chlor bedeuten.

12. Verfahren zur Bekämpfung unerwünschten Pflanzenwachstums, dadurch gekennzeichnet, daß man die unerwünschten Pflanzen und/oder die von unerwünschten Pflanzenwachstum freizuhaltende Fläche mit einer herbizid wirksamen Menge eines Cyclohexenonderivates der Formel I gemäß Anspruch 1 behandelt.

13. Tricarbonylverbindungen der Formel II

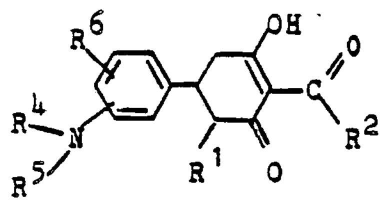

(II),

in der die Substituenten folgende Bedeutung haben:

$R^1$ Wasserstoff, Methoxycarbonyl, Ethoxycarbonyl oder Methyl,

$R^2$ Alkyl mit 1 bis 4 C-Atomen,

$R^4$ Wasserstoff oder Alkyl mit 1 bis 4 C-Atomen,

$R^5$ aliphatisches Acyl mit 2 bis 8 C-Atomen, Formyl, Cycloalkylcarbonyl mit 4 bis 7 C-Atomen, Methoxyalkylcarbonyl, unsubstituiertes oder durch Nitro, Halogen, Alkyl, Alkoxy oder Haloalkyl substituiertes Benzoyl und

$R^6$ Alkyl, Halogen, Hydroxy oder Alkoxy.

**Revendications**

1. Dérivés de cyclohexenone de formule I

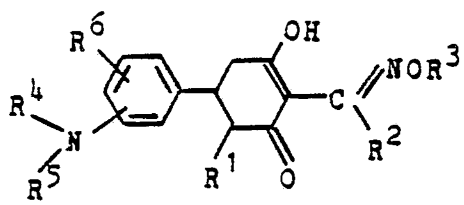

(I),

dans laquelle les substituants ont les significations suivantes:

$R^1$ hydrogène, méthoxycarbonyle, éthoxycarbonyle ou méthyle,

$R^2$ alkyle à 1 à 4 atomes C,

$R^3$ alkyle à 1 à 4 atomes C, alcényle à 3 à 4 atomes C, halogénoalcényle à 3 à 4 atomes C et 1 à 3 substituants halogène ou propargyle,

$R^4$ hydrogène ou alkyle à 1 à 4 atomes C,

$R^2$ acyle aliphatique à 2 à 8 atomes C, formyle, cycloalkylcarbonyle à 4 à 7 atomes C, méthoxyalkylcarbonyle, benzoyle non substitué ou substitué par nitro, halogène, alkyle, alcoxy ou haloalkyle et

$R^6$ alkyle, halogène, hydroxy ou alcoxy,

et sels de ces composés.

2. Dérivés de cyclohexenone de formule I selon la revendication 1, caractérisés par le fait que $R^1$ représente hydrogène.

3. Dérivés de cyclohexenone de formule I selon la revendication 1, caractérisés par le fait que $R^1$ et $R^4$ représentent hydrogène, $R^5$, acétyle ou benzoyle et $R^6$ fluor ou chlore.

4. Procédé de préparation de dérivés de cyclohexenone de formule I selon la revendication 1, caractérisé par le fait que l'on fait réagir un composé tricarbonyle de formule II

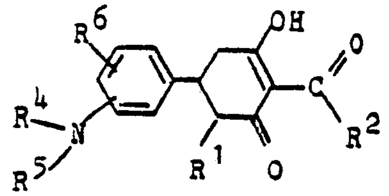

(II),

dans laquelle $R^1$, $R^2$, $R^4$, $R^5$ et $R^6$ ont les significations données dans la revendication 1,

a) avec des composés d'ammonium de formule $R^3O—NH_3Y$, dans laquelle $R^3$ a les significations données dans la revendication 1 et Y représente un anion, dans un diluant inerte, eventuellement en présence d'eau, à une température comprise entre 0 et 80 C, en présence d'une base ou

b) avec des hydroxylamines se trouvant éventuellement en solution aqueuse, de formule $R^3O—NH_2$, dans laquelle $R^3$ a les significations données dans la revendication 1, dans un solvant inerte.

5. Procédé de préparation de dérivés de cyclohexenone de formule $I_a$

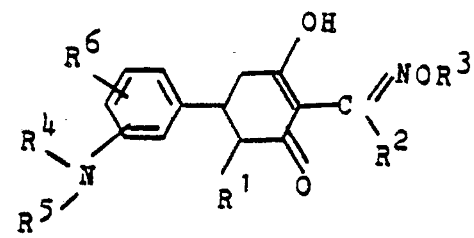

(Ia),

dans laquelle

$R^1$ et $R^4$ représentent hydrogène et

$R^5$ acyle aliphatique à 2 à 8 atomes C, formyle, cycloalkylcarbonyle à 4 à 7 atomes C, méthoxyalkylcarbonyle ou benzoyle éventuellement substitué par nitro, halogène, alkyle, alcoxy ou haloalkyle, et

$R^2$, $R^3$ et $R^6$ ont les significations indiquées dans la revendication 1 pour la formule I, caractérisé par le fait que l'on fait réagir un dérivé de cyclohexenone de formule I, dans laquelle $R^5$ représente hydrogène et $R^1$, $R^2$, $R^3$, $R^4$, et $R^6$ ont les significations sus indiquées, éventuellement dans un système tamponné à une valeur de pH déterminée, éventuellement en présence d'un agent liant d'acide, à une température comprise entre −20 et +150 C avec un composé de formule III

$$R^5Y \qquad \text{(III)}$$

dans laquelle $R^5$ a les significations sus-indiquées et Y est un groupe éliminable.

6. Procédé de préparation de dérivés de cyclohexenone de formule $I_b$

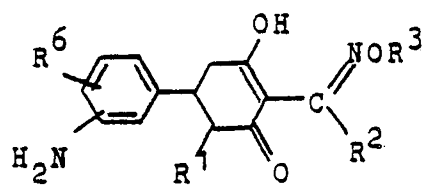

(Ib)

dans laquelle $R^1$, $R^2$ et $R^6$ ont les significations données dans la revendication 1 et $R^3$ représente alkyle à 1 à 4 atomes C, caractérisé par la fait que l'on réduit en composé amino, de manière connue en soi, un composé de formule V

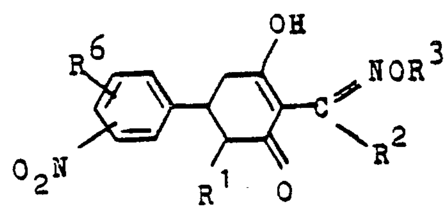

(V)

dans laquelle $R^1$, $R^2$, $R^3$ et $R^6$ ont les significations sus indiquées, avec de l'hydrogène, éventuellement sous pression en présence d'un solvant inerte, éventuellement sous addition de bases et en présence d'un catalyseur d'hydrogénation, à des températures comprises entre 0 et 80°C.

7. Procédé de préparation de composés tricarbonyle de formule $II_a$

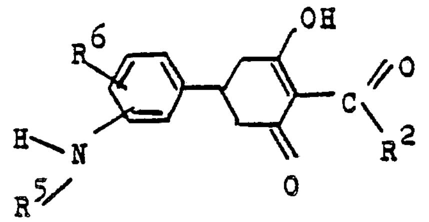

(IIa),

dans laquelle $R^2$, $R^5$ et $R^6$ ont les significations données dans la revendication 1, caractérisé par le fait que l'on réduit en composé amino, de manière connue en soi, un composé de formule VI

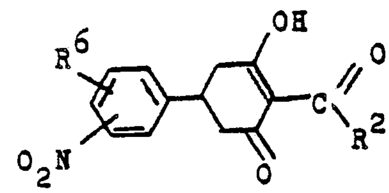

(VI),

dans laquelle $R^2$ et $R^6$ ont les significations sus indiquées, en présence d'un catalyseur d'hydrogénation, à des températures comprises entre 0 et 80 C et on fait réagir ce composé amino, sans autre isolement, avec les composés de formules III et IV.

8. Herbicide, contenant un dérivé de cyclohexenone de formule I selon la revendication 1.

9. Herbicide, contenant un additif inerte et un dérivé de cyclohexenone de formule I selon la revendication 1.

10. Herbicide selon la revendication 9, caractérisé par le fait qu'il contient 0,1 à 95% en poids de dérivé de cyclohexenone de formule I.

11. Herbicide selon la revendication 9, caractérisé par le fait qu'il contient un dérivé de cyclohexenone de formule I, $R^1$ et $R^4$ représentant hydrogène, $R^5$, acétyle ou benzoyle et $R^6$, fluor ou chlore.

12. Procédé de lutte contre une croissance des plantes indésirables, caractérisé par le fait que l'on traite les plantes indésirables et/ou les surfaces à maintenir exemptes d'une croissance des plantes indésirables, avec une quantité efficace du point de vue herbicide d'un dérivé de cyclohexenone de formule I, selon la revendication 1.

13. Composés tricarbonyle de formule II

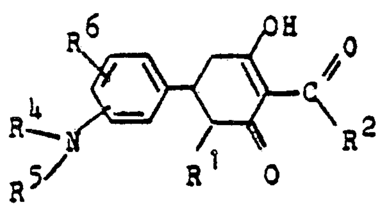

(II),

dans laquelle les substituants ont les significations suivantes:

$R^1$ hydrogène, méthoxycarbonyle, éthoxycarbonyle ou méthyle,

$R^2$, alkyle à 1 à 4 atomes C,

$R^4$, hydrogène ou alkyle à 1 à 4 atomes C,

$R^5$ acyle aliphatique à 2 à 8 atomes C, formyle, cycloalkylcarbonyle à 4 à 7 atomes C, méthoxyalkylcarbonyle, benzoyle non substitué ou substitué par nitro, halogène, alkyle, alcoxy ou haloalkyle et

$R^6$, alkyle, halogène, hydroxy ou alcoxy.

**Claims**

1. A cyclohexenone derivative of the formula I

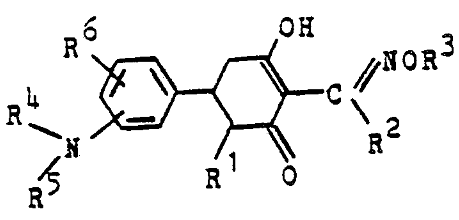

(I),

where

$R^1$ is hydrogen, methoxycarbonyl, ethoxycarbonyl or methyl,

$R^2$ is alkyl of 1 to 4 carbon atoms,

$R^3$ is alkyl of 1 to 4 carbon atoms, alkenyl of 3 to 4 carbon atoms, haloalkenyl of 3 to 4 carbon atoms which possesses 1 to 3 halogen substituents, or propargyl,

$R^4$ is hydrogen or alkyl of 1 to 4 carbon atoms,

$R^5$ is aliphatic acyl of 2 to 8 carbon atoms, formyl, cycloalkylcarbonyl of 4 to 7 carbon atoms, methoxyalkylcarbonyl, or benzoyl which is unsubstituted or substituted by nitro, halogen, alkyl, alkoxy or haloalkyl and

$R^6$ is alkyl, halogen, hydroxyl or alkoxy, and

a salt of this compound.

2. A cyclohexenone derivative of the formula I as claimed in claim 1, where $R^1$ is hydrogen.

3. A cyclohexenone derivative of the formula I as claimed in claim 1, where $R^1$ and $R^4$ are hydrogen, $R^5$ is acetyl or benzoyl, and $R^6$ is fluorine or chlorine.

4. A process for the preparation of a cyclohexenone derivative of the formula I as claimed in claim 1, wherein a tricarbonyl of the formula II

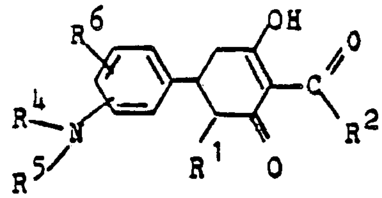

(II),

where $R^1$, $R^2$, $R^4$, $R^5$ and $R^6$ have the meanings given in claim 1, is reacted

a) with an ammonium compound of the formula $R^3O—NH_3Y$, where $R^3$ has the meanings given in claim 1 and Y is an anion, in an inert diluent, in the presence or absence of water, at from 0° to 80°C, and in the presence of a base, or

b) with a hydroxylamine — if desired, in aqueous solution — of the formula $R^3O—NH_2$, where $R^3$ has the meanings given in claim 1, in an inert solvent.

5. A process for the preparation of a cyclohexenone derivative of the formula Ia

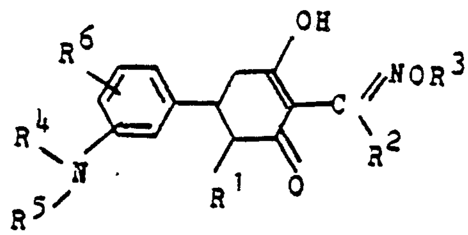

(Ia),

where

$R^1$ and $R^4$ are each hydrogen,

$R^5$ is aliphatic acyl of 2 to 8 carbon atoms, formyl, cycloalkylcarbonyl of 4 to 7 carbon atoms, methoxyalkylcarbonyl, or benzoyl which is unsubstituted or substituted by nitro, halogen, alkyl, alkoxy, haloalkyl, and

$R^2$, $R^3$ and $R^6$ have the meanings given for formula I in claim 1, wherein a cyclohexenone derivative of the formula I where $R^5$ is hydrogen and $R^1$, $R^2$, $R^3$, $R^4$ and $R^6$ have the above meanings, is reacted, if desired in a system buffered to a certain pH value, in the presence or absence of an acid acceptor and at from −20° to +150°C, with a compound of the formula III

$$R^5Y \quad \text{(III)},$$

where $R^5$ has the above meanings and Y is a leaving group.

6. A process for the preparation of a cyclohexenone derivative of the formula Ib

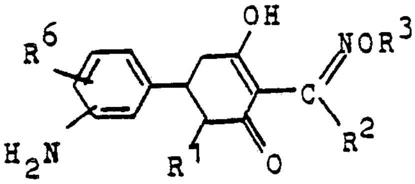

(Ib)

where $R^1$, $R^2$ and $R^6$ have the meanings given in claim 1 and $R^3$ is alkyl of 1 to 4 carbon atoms, wherein a compound of the formula V

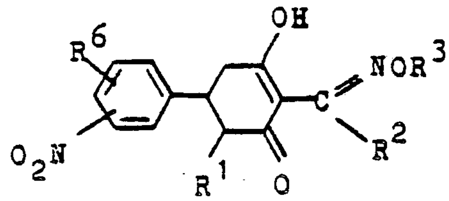

(V)

where $R^1$, $R^2$, $R^3$ and $R^6$ have the above meanings, is reduced to the amino compound in conventional manner with hydrogen, at atmospheric or superatmospheric pressure, in the presence of an inert solvent, with or without the addition of a base, in the presence of a hydrogenation catalyst and at from 0° to 80°C.

7. A process for the preparation of a tricarbonyl compound of the formula IIa

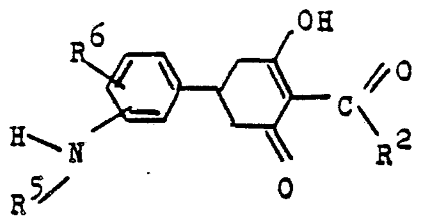

(IIa),

where $R^2$, $R^5$ and $R^6$ have the meanings given in claim 1, wherein a compound of the formula VI

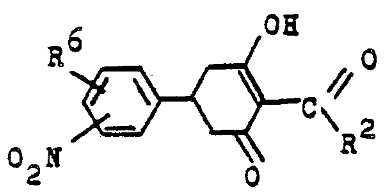

(VI),

where $R^2$ and $R^6$ have the above meanings, is reduced in conventional manner to the amino compound in the presence of a hydrogenation catalyst at from 0° to 80°C, and the amino compound is reacted without further isolation to give a compound of the formula III or IV.

8. A herbicide containing a cyclohexenone derivative of the formula I as claimed in claim 1.

9. A herbicide containing inert additives and a cyclohexenone derivative of the formula I as claimed in claim 1.

10. A herbicide as claimed in claim 9, containing from 0.1 to 95% by weight of a cyclohexenone derivative of the formula I.

11. A herbicide as claimed in claim 9, containing a cyclohexenone derivative of the formula I in which $R^1$ and $R^4$ are hydrogen, $R^5$ is acetyl or benzoyl, and $R^6$ is fluorine or chlorine.

12. A process for containing the growth of unwanted plants, wherein the unwanted plants and/or the area to be kept free from unwanted plant growth are treated with a herbicidally effective amount of a cyclohexenone derivative of the formula I as claimed in claim 1.

13. A tricarbonyl compound of the formula II

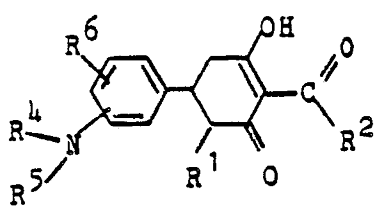

(II),

where

$R^1$ is hydrogen, methoxycarbonyl, ethoxycarbonyl or methyl,

$R^2$ is alkyl of 1 to 4 carbon atoms,

$R^4$ is hydrogen or alkyl of 1 to 4 carbon atoms,

$R^5$ is aliphatic acyl of 2 to 8 carbon atoms, formyl, cycloalkylcarbonyl of 4 to 7 carbon atoms, methoxyalkylcarbonoyl, or benzoyl which is unsubstituted or substituted by nitro, halogen, alkyl, alkoxy or haloalkyl, and

$R^6$ is alkyl, halogen, hydroxyl or alkoxy.